(19) Europäisches Patentamt / European Patent Office / Office européen des brevets

(11) **EP 2 935 587 B1**

(12) **FASCICULE DE BREVET EUROPEEN**

(45) Date de publication et mention
de la délivrance du brevet:
**14.03.2018 Bulletin 2018/11**

(21) Numéro de dépôt: **13811573.8**

(22) Date de dépôt: **20.12.2013**

(51) Int Cl.:
*C12N 15/115* *(2010.01)*       *A61K 31/7088* *(2006.01)*
*A61P 17/00* *(2006.01)*       *A61Q 19/02* *(2006.01)*

(86) Numéro de dépôt international:
**PCT/EP2013/077801**

(87) Numéro de publication internationale:
**WO 2014/102213 (03.07.2014 Gazette 2014/27)**

(54) **APTAMERES INHIBITEURS DE L'ACTIVITE ENZYMATIQUE DE LA TYROSINASE**

APTAMERE ZUR HEMMUNG DER ENZYMATISCHEN AKTIVITÄT VON TYROSINASE

APTAMERS INHIBITING THE ENZYMATIC ACTIVITY OF TYROSINASE

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **24.12.2012 FR 1262754**

(43) Date de publication de la demande:
**28.10.2015 Bulletin 2015/44**

(73) Titulaires:
• **LVMH RECHERCHE**
**45800 St. Jean de Braye (FR)**
• **Institut National de la Santé et de la
Recherche Médicale (INSERM)
75013 Paris (FR)**

(72) Inventeurs:
• **CAUCHARD, Jean-Hubert
45560 SAINT DENIS EN VAL (FR)**
• **KURFURST, Robin
45800 Saint Jean de Braye (FR)**
• **SCHNEBERT, Sylvianne
45160 Olivet (FR)**
• **DAUSSE, Eric
33000 Bordeaux (FR)**
• **TOULME, Jean-Jacques
33310 Lormont (FR)**

(74) Mandataire: **Regimbeau
20, rue de Chazelles
75847 Paris Cedex 17 (FR)**

US-A1- 2009 202 458

• **HIDEYA ANDO ET AL: "Approaches to Identify
Inhibitors of Melanin Biosynthesis via the Quality
Control of Tyrosinase", JOURNAL OF
INVESTIGATIVE DERMATOLOGY, vol. 127, no. 4,
11 janvier 2007 (2007-01-11), pages 751-761,
XP055079312, ISSN: 0022-202X, DOI:
10.1038/sj.jid.5700683**
• **N. JAANSON ET AL: "332 The Anti-tyrosinase
Monoclonal Antibody is Effective in Treating Mice
Bearing Syngenic Melanoma", EUROPEAN
JOURNAL OF CANCER, vol. 48, octobre 2012
(2012-10), pages 101-102, XP055079232, ISSN:
0959-8049, DOI: 10.1016/S0959-8049(12)72130-5**
• **SANG MI AN ET AL: "Inhibition of melanogenesis
by tyrosinase siRNA in human melanocytes",
BMB REPORTS, vol. 42, no. 3, 31 mars 2009
(2009-03-31), pages 178-183, XP055050489,**
• **LIN SHI-LUNG ET AL: "CHAPTER 4: Recent
Application of Intronic MicroRNA Agents in
Cosmetics", CURRENT PERSPECTIVES IN
MICRORNAS (MIRNA), SPRINGER
NETHERLANDS, NL, 2008, pages 51-72,
XP009136584, ISBN: 978-1-4020-8532-1**
• **DUPONT ET AL: "Nucleic Acid Aptamers Against
Proteases.", CURRENT MEDICINAL CHEMISTRY,
vol. 18, 1 septembre 2011 (2011-09-01), pages
4139-4151, XP055009453, ISSN: 0929-8673**

(56) Documents cités:
**WO-A2-01/58918       WO-A2-2009/129497**

EP 2 935 587 B1

- MISSAILIDIS S ET AL: "44 Development of diagnostic and therapeutic aptamers against enzymes crucial for tumour development and metastasis", EUROPEAN JOURNAL OF CANCER. SUPPLEMENT, PERGAMON, OXFORD, GB, vol. 8, no. 5, juin 2010 (2010-06), pages 11-12, XP027105283, ISSN: 1359-6349 [extrait le 2010-06-01]
- M TAREQ HASSAN KHAN ET AL: 'Modelling of the mushroom tyrosinase and its molecular dynamic (MD) simulations experiments in room and elevated temperatures and the docking interactions with nucleotides' MINERVA BIOTECNOLOGICA, [en ligne] 01 Décembre 2006, TORINO, page 171, XP055324008 Extrait de l'Internet: <URL:http://www.minervamedica.it/en/getfree pdf/n%2BM%2FwT51IHQd%2BEz0zkFa%2BGcl2 JQov21 lfYhll%2BZHe47L4KsYH8zDynw1w9jLcxdnsprc xne1 xr5BWAchVbfvbw%3D%3D/R04Y2006N04A0171 .pdf>

SEQUENCE LISTING

<110> INSTITUT NATIONAL DE LA SANTE ET DE LA RECHERCHE MEDICALE (INSERM)
L V M H RECHERCHE

<120> APTAMERES INHIBITEURS DE L'ACTIVITE ENZYMATIQUE DE LA TYROSINASE

<130> 364479D29495

<160> 12

<170> PatentIn version 3.5

<210> 1
<211> 77
<212> DNA
<213> artificial

<220>
<223> Aptamere

<400> 1

```
gcctgttgtg agcctcctgt cgaacgcaat gggcgcagat tggaaggcct agcattgagc      60

gtttattctt gtctccc                                                     77
```

<210> 2
<211> 37
<212> DNA
<213> artificial

<220>
<223> Aptamere

<400> 2
```
gcctcctgtc gaacgcaatg ggcgcagatt ggaaggc 37
```

<210> 3
<211> 30
<212> DNA
<213> artificial

<220>
<223> Aptamere

<400> 3
```
ccctgtcgaa cgcaatgggc gcagattggg      30
```

<210> 4
<211> 22
<212> DNA
<213> artificial

<220>
<223> Aptamere

<400> 4
```
cccgaacgca atgggcgcag gg 22
```

<210> 5
<211> 11
<212> DNA
<213> artificial

<220>
<223> Aptamere

<400> 5
```
cgcaatgggc g 11
```

<210> 6
<211> 30
<212> DNA
<213> artificial

<220>
<223> Aptamere

<400> 6
cgcaatgggc gcagattgga aggcctagca        30

<210> 7
<211> 24
<212> DNA
<213> artificial

<220>
<223> Amorce P5

<400> 7
gcctgttgtg agcctcctgt cgaa 24

<210> 8
<211> 23
<212> DNA
<213> artificial

<220>
<223> Aptamere

<400> 8
ttgagcgttt attcttgtct ccc        23

<210> 9
<211> 23
<212> DNA
<213> artificial

<220>
<223> Amorce P3

<400> 9
gggagacaag aataaacgct caa 23

<210> 10
<211> 40
<212> DNA
<213> artificial

<220>
<223> Aptamere

<400> 10
tcgaacgcaa tgggcgcaga ttggaaggcc tagcattgag        40

<210> 11
<211> 36
<212> DNA
<213> artificial

<220>
<223> Aptamere

<400> 11

gtcgaacgca atgggcgcag attggaaggc ctagca 36

<210> 12
<211> 22
<212> DNA
<213> artificial

<220>
<223> Aptamere

<220>
<221> stem_loop
<222> (1)..(22)
<223> tige entre les nucléotides 1 à 3 et 20 à 22
boucle entre les nucléotides 4 à 6 et 18 à 19
tige entre les nucléotides 7 à 9 et 15 à 17
boucle entre les nucléotides 10 à 14

<220>
<221> misc_feature
<222> (1)..(22)
<223> n is a, c, g, or t

<400> 12
nnnnnnnnnn nnnnnnnnnn nn 22

## Description

L'invention concerne le domaine des applications dermocosmétiques et dermatologiques dépigmentantes ou éclaircissantes.

Chez l'homme, la pigmentation résulte de la synthèse et de la distribution des pigments mélaniques dans la peau, les follicules pileux ou les yeux. La pigmentation est génétiquement prédéfinie. Les gènes contrôlant la pigmentation agissent directement sur la cellule pigmentaire, ou indirectement sur l'environnement de cette dernière, notamment les kératinocytes.

De nombreux gènes agissant sur la cellule pigmentaire ont été identifiés et caractérisés, dont les produits sont des protéines contrôlant la synthèse des mélanines, des protéines de structure des mélanosomes, ou contrôlant la biogénèse et le transport des mélanosomes.

Parmi les facteurs exogènes stimulant la mélanogenèse, les rayons ultraviolets viennent au premier rang. L'inflammation et les stress oxydatifs tels par exemple ceux déclenchés par la pollution stimulent la synthèse de mélanine. Ces stress agissent directement sur les mélanocytes et aussi indirectement par activation des kératinocytes qui vont alors libérer des facteurs tels que des cytokines ou des hormones. Ces facteurs, tels que les interleukines, l'alpha MSH, l'endothéline 1, les prostaglandines E2, vont déclencher la cascade de réactions conduisant à la synthèse de la mélanine. Ces stimulations répétées associées à des désordres de la jonction dermo-épidermique sont à l'origine de la formation d'une pigmentation cutanée hétérogène qui se manifeste par exemple par l'apparition d'éphélides, de mélasmas ou encore des lentigines.

Provoquées par un excès de mélanine dans la peau, les hyperpigmentations sont la conséquence de facteurs intrinsèques (facteurs endocriniens, métaboliques, génétiques) ou extrinsèques (agents photosensibilisants, physiques, chimiques, inflammatoires...) parfois étroitement liés et difficiles à préciser. La plupart des hyperpigmentations résulte d'une suractivité mélanocytaire conduisant à une surproduction de mélanine. Ces hyperpigmentations apparaissent au niveau du visage ou des mains et se manifestent par des zones ou des taches plus foncées ou plus colorées conférant à la peau une hétérogénéité pigmentaire.

Tous les pigments mélaniques proviennent d'une voie de synthèse commune, mettant en jeu un acide aminé, la tyrosine et une enzyme-clé, la tyrosinase qui peut être libre ou sous la forme d'un complexe tyrosinase / tyrosine -related protein-1. La tyrosinase transforme la tyrosine en DOPA, puis en DOPAquinone. À partir de la DOPAquinone deux voies sont possibles, l'une conduit à la phaeomélanine, l'autre à l'eumélanine. Dans la voie des eumélanines, la dopaquinone conduit à la formation de dopachrome. Le dopachrome via la formation de dihydroxyindole ou de dihydroxyindolecarboxyl conduit à la formation d'eumélanine. La tyrosinase est l'enzyme clef de cette cascade de réactions qui conduit à la formation de mélanine en catalysant seule ou en complexe avec la tyrosinase-related protein 1, plusieurs réactions de cette cascade.

Ainsi, afin de prévenir les hyperpigmentations et inhiber la mélanogenèse, il est important d'inhiber voire de supprimer l'activité de la tyrosinase dans la peau.

Les mélanocytes expriment la tyrosinase. Ainsi, il a été envisagé de développer des inhibiteurs spécifiques de la tyrosinase.

Composés chimiques, naturels, ou synthétiques, les agents régulateurs de la pigmentation cutanée peuvent agir selon plusieurs types de mécanismes :

☐ Soit en bloquant la stimulation des mélanocytes par les kératinocytes
☐ Soit en bloquant la voie de synthèse de la mélanine à un niveau donné
☐ Soit en bloquant le transfert des mélanosomes
☐ Soit en diminuant le nombre de mélanocytes.

Parmi ces mécanismes d'action, les agents dépigmentants peuvent être des inhibiteurs de l'activité enzymatique de la tyrosinase.

Les premiers actifs de régulation de la pigmentation cutanée, inhibiteurs de l'activité enzymatique de la tyrosinase utilisés, ont été les sels de mercure, le chlorure mercurique, le chlorure d'amine mercure. Le mercure entrant en compétition avec le cuivre, inhibe l'activité de la tyrosinase, supprimant les deux premières phases de la synthèse de la mélanine. Cependant, le mercure est une substance très toxique. L'hydroquinone, des éthers de l'hydroquinone ou des dérivés ont été proposés. Elle agit par inhibition compétitive de la tyrosinase. Cependant, elle induit des altérations mitochondriales et des effets secondaires comme des irritations ou des allergies. Des études toxicologiques réalisées sur ces substances ont montré un risque d'inhibition de la spermatogenèse et de cancer cutané.

M Tareq Hassan Khan et al., (Minerva Biotecnologica, vol. 18, décembre 2006, pages 171-179) décrit un aptamère ADN virtuel susceptible d'interagir avec une tyrosinase de champignon. L'utilisation topique de substances dépigmentantes efficaces et inoffensives est particulièrement recherchée en cosmétique et en dermatologie. On utilise notamment ces substances pour traiter des hyperpigmentations régionales par hyper-activité mélanocytaire telles que les mélasmas idiopathiques, les hyperpigmentations localisées par hyper-activité mélanocytaire telles que les taches pigmentaires dites lentigos solaires et lentigos séniles, les hyperpigmentations accidentelles telles que la photosensibilisation ou la cicatrisation post-lésionnelle, mais aussi pour des leucodermies telles que le vitiligo. Dans ces derniers cas, à défaut de pouvoir repigmenter la peau, on atténue la pigmentation de la périphérie des zones dépigmentées pour donner à la peau une couleur plus homogène.

Des substances dépigmentantes sont également utilisées en tant qu'agents de blanchiment de la peau, en particulier celles désignées plus haut, qui sont très réactives aux UV, pour éclaircir leur teint, notamment celui de leur visage et de leurs mains, afin de conserver une couleur de peau la plus claire possible ou encore réduire les effets pigmentants des rayons UV.

Le but de l'invention est de résoudre les problèmes mentionnés ci-dessus des techniques de l'art antérieur et de proposer une composition cosmétique particulièrement avantageuse pour inhiber l'activité enzymatique de la tyrosinase.

Résumé de l'invention

L'invention a pour objet un nouvel inhibiteur de la tyrosinase efficace par voie topique pour inhiber la mélanogénèse, stable dans un milieu cosmétiquement acceptable et non irritant, non toxique et/ou non allergisant.

L'invention a encore pour objet un nouvel agent dépigmentant agissant sur le processus de la mélanogénèse, destiné d'une part, dans le cas d'une pigmentation sensiblement homogène, au blanchiment de la peau, des poils ou des cheveux, c'est-à-dire à diminuer leur pigmentation, et d'autre part, à lutter contre l'hyperpigmentation cutanée à savoir lorsque la peau présente une hétérogénéité de pigmentation.

Les inventeurs ont observé de façon surprenante qu'un aptamère d'ADN résistant aux nucléases capable de se lier spécifiquement à la tyrosinase et inhibant l'activité tyrosinase remplissait tous ces critères. En particulier, il est capable de pénétrer dans des cellules de peau.

Pour isoler des aptamères anti-tyrosinase, les inventeurs ont utilisé une banque de séquences oligonucléotidiques phosphodiester (même composition que l'ADN naturel). La sélection a été faite de façon dirigée pour identifier, isoler et caractériser des séquences pouvant interagir spécifiquement avec la tyrosinase.

Ces aptamères ont ensuite été testés pour leur capacité à inhiber l'activité de la tyrosinase.

Un premier objet de l'invention concerne donc un aptamère d'ADN comprenant la séquence SEQ ID NO: 5, de préférence résistant aux nucléases, capable d'inhiber l'activité enzymatique de la tyrosinase et la conversion de la tyrosine en L-DOPA et Dopaquinone .

Un deuxième objet de l'invention concerne l'utilisation d'un aptamère selon l'invention pour inhiber l'activité enzymatique de la tyrosinase.

Un troisième objet de l'invention concerne une composition cosmétique ou pharmaceutique comprenant en tant que principe actif un aptamère selon l'invention en une quantité suffisante pour inhiber la tyrosinase et un ou plusieurs excipients pharmaceutiquement acceptables.

Un quatrième objet de l'invention concerne l'utilisation cosmétique d'un aptamère selon l'invention.

Un cinquième objet de l'invention concerne un aptamère selon l'invention en tant que médicament. La spécification décrit également un procédé de sélection d'un aptamère selon l'invention comprenant les étapes suivantes :

- Sélection dans une banque d'ADN d'aptamères par la méthode SELEX contre le site catalytique de la tyrosinase,
- évaluation du potentiel à inhiber l'activité enzymatique de la tyrosinase des aptamères identifiés à l'étape précédente,
- clonage et séquençage des aptamères ainsi sélectionnés.

**Résumé des figures**

    **Figure 1 : Structures secondaires prédites de l'aptamère T10.1 et ses versions tronquées.**
    **Figure 2 : Sensorgramme du complexe aptamère T10.1 -Tyrosinase**
    **Figure 3 : Mesure de la modulation de l'activité tyrosinase par différents aptamères issus du tour 10.**
    **Figure 4 : Courbe (P) = f(t), relation entre l'apparition du produit P et le temps t**

**Description détaillée de l'invention**

Un premier objet de l'invention concerne donc un aptamère d'ADN comprenant la séquence SEQ ID NO: 5, préférentiellement résistant aux nucléases, capable d'inhiber l'activité enzymatique de la protéine tyrosinase de conversion de la tyrosine en L-DOPA.

On entend par « aptamère » une molécule d'ADN ou d'ARN ligand spécifique et de forte affinité d'une protéine. Cette acception comprend par conséquent les aptamères « naturels » et les analogues modifiés chimiquement.

Les aptamères sont sélectionnés par alternance sélection/amplification qui permet de diriger l'évolution de la population selon un mode darwinien : dans la population, les molécules les plus aptes sont sélectionnés, d'où le nom « aptamères » donné aux oligonucléotides présentant le caractère désiré, issus de la sélection. Les techniques classiques de génie génétique (clonage, séquençage, expression) permettent aisément d'identifier individuellement ces aptamères, de les caractériser et de les produire ensuite en quantité.

La sélection des aptamères peut être réalisée par un protocole optimisé de sélection in vitro, connu sous le nom de "SELEX » (Systemic Evolution of Ligands by Exponential enrichment) (WO 91/19813).

Le procédé SELEX permet de générer des ligands de très haute affinité et spécificité en grande quantité. Cette approche repose sur la mise en contact de la molécule cible avec une banque de ligands potentiels. Un système de cycles de désorption/sélection permet d'enrichir la population des ligands interagissant le plus spécifiquement avec la molécule cible. La population obtenue en final est ensuite isolée et caractérisée permettant sa resynthèse à grande échelle.

Bien que le procédé SELEX ait été établi comme une technique générale pour sélectionner des aptamères, il n'est néanmoins ni prévisible ni standardisé pour n'importe quelle cible. Au contraire, le procédé SELEX doit être optimisé et adapté pour chaque cible particulière. Le procédé SELEX n'est pas garanti pour toute cible.

Plusieurs facteurs sont importants pour une sélection d'aptamères. Par exemple, la molécule cible doit être stable et facilement reproductible à chaque cycle de SELEX, parce que le procédé SELEX implique plusieurs cycles de liaison, sélection, et amplification. En outre, les acides nucléiques qui montrent une liaison spécifique à la cible doivent être présents dans la banque initiale. Ainsi, il est nécessaire de produire une banque initiale d'acides nucléiques très diversifié.

La sélection d'aptamères en utilisant le procédé SELEX est imprévisible. Même si tous les facteurs sont optimums pour la sélection d'aptamères, le procédé SELEX ne permet pas toujours d'obtenir des aptamères viables pour chaque cible.

La banque initiale de candidats est composée de séquences ADN synthétisées chimiquement, chacune comportant une région variable longue de n nucléotides flanquée, en 3' et en 5', de régions fixes identiques pour tous les candidats de la banque. Ces régions fixes permettent la manipulation de la partie centrale au cours du SELEX, notamment l'amplification par PCR. La taille de la portion variable dicte la diversité de la banque qui est égale à $4^n$ puisque chaque position peut être occupée par l'un des quatre nucléotides A, T, G ou C. Pour des fenêtres de grande taille, on atteint des complexités gigantesques : pour $n = 50$ la diversité théorique est de $4^{50}$ soit $10^{30}$, une valeur inaccessible en pratique puisqu'elle correspond à plus de $10^5$ tonnes pour une banque dans laquelle chaque séquence est représentée une fois. La limite expérimentale se situe autour de $10^{15}$ séquences différentes, soit celle d'une banque dans laquelle tous les candidats possédant une région variable de 25 nucléotides sont représentés. Si l'on choisit de manipuler une banque comportant une fenêtre de 30 nucléotides dont la diversité théorique est d'environ $10^{18}$, on n'explorera donc que $1/1000^e$ des possibilités. Dans la pratique, cela est généralement suffisant pour obtenir des aptamères possédant les propriétés voulues. D'ailleurs, les polymérases utilisées étant infidèles et introduisant des erreurs à un taux de l'ordre de $10^{-4}$, elles contribuent à enrichir significativement la diversité du pool de séquences tout au long du processus SELEX : un candidat sur 100 sera modifié à chaque cycle d'amplification pour une banque avec une région aléatoire de 100 nucléotides, conduisant donc à l'apparition de $10^{13}$ nouveaux candidats pour la banque totale.

La sélection s'opère à chaque tour, par séparation physique entre molécules associées à la cible et molécules libres. De multiples techniques peuvent être mises en oeuvre (chromatographie, rétention sur filtre, électrophorèse...). Les conditions de la sélection sont ajustées (concentration relative cible/candidats, concentration ionique, température, lavage...) pour qu'une compétition intervienne entre les candidats pour fixation à la cible. D'une façon générale, la stringence est augmentée au fil des tours de façon à favoriser la capture des candidats les plus affins. Par ailleurs, une contre-sélection est effectuée pour éliminer les candidats qui reconnaissent le support (filtre, billes...)

Les oligonucléotides sont des oligo-anions, chaque motif unitaire possédant une charge à pH neutre, des sites donneurs/accepteurs de liaison hydrogène et un hétérocycle aromatique (la base nucléique) pouvant générer des interactions d'empilement. Ces oligomères se replient suite à la formation de paires de bases, pour générer des structures secondaires et tertiaires. La banque de séquences initiales est donc une banque de formes tridimensionnelles, chacune correspondant à une distribution de motifs, susceptibles d'engager des interactions électrostatiques, de donner naissance à des liaisons H, etc. La sélection revient à identifier dans la banque la forme adaptée à la cible, c'est-à-dire celle permettant le plus grand nombre d'interactions, la formation du complexe aptamère-cible, le plus stable. Pour des cibles de petite taille (colorants, antibiotiques...) les aptamères identifiés sont caractérisés par des constantes d'équilibre de dissociation de l'ordre du micromolaire tandis que pour des cibles protéiques les $K_d$ inférieures à $10^{-9}$ M ne sont pas rares.

Des cibles très variées ont été visées pour générer des aptamères : des acides aminés, des peptides, des protéines ou des enzymes mais aussi des structures complexes comme des virus intacts ou des cellules vivantes.

La propriété la plus remarquable des aptamères est la spécificité des interactions engagées avec leur ligand, ce qui en fait des agents de premier plan pour la reconnaissance d'une cible.

Les aptamères peuvent être des oligodésoxy- (ADN) ou des oligoribo-nucléotides (ARN). Dans ce dernier cas, la première étape du SELEX consiste en une transcription de la banque initiale ADN, la partie fixe 5' des candidats comportant une séquence promotrice. Après sélection, les candidats sont convertis en ADN par transcription inverse avant d'être amplifiés. Des aptamères ARN et ADN ayant des caractéristiques comparables ont été sélectionnés contre une même cible. De plus les deux composés sont inhibiteurs compétitifs l'un de l'autre suggérant un recouvrement des sites d'interaction. Cela a des conséquences majeures pour la réalisation d'aptamères chimiquement modifiés.

Le développement de l'approche antisens a conduit à la synthèse de très nombreux analogues dont certains par exemple confèrent à l'oligomère une résistance aux nucléases, une propriété utile pour une utilisation dans un environnement biologique (milieu de culture cellulaire ou *in vivo*). Des modifications de la liaison phosphodiester, du sucre ou du squelette phosphate-sucre tels les dérivés 2'-O-méthyle, acide nucléique « *locked* » ou borano-phosphate conduisent à des oligomères résistants aux nucléases. Cette propriété peut présenter un intérêt pour les aptamères. Cependant, comme mentionné précédemment, le changement complet de structure chimique *a posteriori* d'un aptamère sélectionné sous forme ARN ou ADN conduit généralement à une diminution voire à une perte des propriétés pour lesquelles il a été sélectionné. Cela ne signifie pas qu'il ne soit pas possible d'introduire des modifications à certaines positions. Mais il convient d'identifier les positions auxquelles les modifications sont tolérées. Cela peut être réalisé par essai de variants ponctuels ou par une approche systématique dite d'interférence chimique qui est une variante de la cartographie par empreinte.

Il est bien sûr préférable d'opérer directement la sélection d'oligonucléotides non naturels. Cela suppose que les nucléosides triphosphates modifiés soient efficacement incorporés et les matrices modifiées correctement lues par les polymérases utilisées au cours du SELEX. Un très petit nombre d'analogues satisfont aux exigences. Pour ce qui est des dérivés qui confèrent une résistance aux nucléases, les possibilités se limitent aux analogues phosphorothiate, boranophosphate, 2'-méthyle, 2'amino- ou 2'fluoro-pyrimidine, ces derniers étant de loin les plus utilisés. Les aptamères identifiés dans ce cas ont des nucléosides pyrimidiques modifiés et des résidus puriques non modifiés (2'hydroxyle). Ces molécules présentent une résistance accrue aux nucléases. Si nécessaire, des résidus puriques modifiés peuvent être introduits *a posteriori,* comme indiqué précédemment. Par ailleurs, il est possible de sélectionner des oligonucléotides comportant des substituants sur la position C(5) des pyrimidines ou N(7), C(8) des purines. Cela n'a pas d'effet sur la sensibilité aux nucléases, mais permet d'ajouter de nouvelles fonctionnalités (hydrophobicité, photoréactivité...).

Une approche très différente concerne l'utilisation d'isomères optiques. Les acides nucléiques naturels sont les isomères D. Les analogues L sont résistants aux nucléases mais ne peuvent être produits par les polymérases. Selon les lois de l'isomérie optique, un aptamère en série L formera avec sa cible C un complexe ayant les mêmes caractéristiques que le complexe formé par l'isomère en série D et l'énantiomère C' de la cible C. Dès lors, si l'on peut synthétiser chimiquement le composé C', on l'utilisera pour réaliser la sélection d'un aptamère naturel (D). Une fois identifié, cet aptamère sera synthétisé chimiquement en série L. Cet aptamère L sera un ligand de la cible naturelle C.

Une autre approche, récemment décrite comme SELEX bidimensionnel, fait intervenir simultanément la sélection *in vitro* d'oligonucléotides et la chimie combinatoire dynamique (CCD), c'est-à-dire la mise en oeuvre d'une réaction réversible entre certains groupes de l'oligonucléotide (des groupements amines) et une banque de composés aldéhydiques. La réaction aboutit à la production d'oligonucléotides imines qui sont sélectionnés sur les mêmes principes que pour le SELEX classique. Il a ainsi été possible d'identifier pour une cible ARN en épingle des aptamères modifiés qui diffèrent des aptamères naturels.

Au contraire des modifications de squelette qui peuvent altérer la structure et qui nécessitent que des précautions soient prises avant d'être introduites sous peine de perdre les propriétés d'interaction de l'aptamère avec sa cible, il est possible de conjuguer différents groupements à l'une des extrémités 3' ou 5 de l'oligonucléotide pour le convertir en outil, sonde ou capteur sans perturber ses caractéristiques. Cette versatilité constitue d'ailleurs un intérêt important des aptamères, notamment dans des perspectives diagnostiques.

L'expression « analogue d'aptamère » s'entend ici de l'une ou plusieurs des modifications décrites ci-dessus. De préférence, l'aptamère selon l'invention est capable de se lier avec une forte affinité à la tyrosinase.

On entend par « forte affinité » au sens de la présente invention une interaction spécifique de l'aptamère avec cible et avec une constante de dissociation suffisamment faible pour permettre l'inhibition significative de l'activité catalytique de l'enzyme. L'aptamère selon l'invention comprend au moins la séquence SEQ ID NO :5. Préférentiellement, l'aptamère selon l'invention comprend au moins une séquence choisie dans le groupe consistant en SEQ ID NO :1, SEQ ID NO :2, SEQ ID NO :3, SEQ ID NO :4 et SEQ ID NO :6.

La séquence SEQ ID NO :1 a été sélectionnée par la méthode SELEX.

Les séquences SED ID NO :2 à 5 sont des dérivés raccourcis de SEQ ID NO :1.

La séquence SEQ ID NO :6 correspond à la partie de SEQ ID NO :1 sans les amorces.

Le tableau 1 suivant résume ces séquences.

**Tableau 1 : Séquences des aptamères d'ADN raccourcis issues de l'aptamère T10.1 représenté à 42% dans le tour 10.**

| | | |
|---|---|---|
| T10.1 SEQ ID NO :1 | *GCCTGTTGTGAGCCTCCTGTCGAA*CGCAATGGGCGCAGATTGGAAGGCCTAGC*ATTGAGCGTTTATTCTTGTCTCCC*<br><br>SEQ ID NO:7    SEQ ID NO:6    SEQ ID NO:8 | SEQ ID NO: 1 |
| T10.1A SEQ ID NO :2 | *GCCTCCTGTCGAACGCA*ATGGGCGCAGATTGGAAGGC | SEQ ID NO:2 |
| T10.1B SEQ ID NO :3 | CCCTGTCGAACGCAATGGGCGCAGATTGGG | SEQID NO:3 |
| T10.1C SEQ ID NO :4 | *CCCGAA*CGCAATGGGCGCAGGG | SEQ ID NO:4 |
| T10.1D SEQ ID NO :5 | CGCAATGGGCG | SEQ ID NO:5 |

EP 2 935 587 B1

Encore préférentiellement, l'aptamère selon l'invention comprend au moins 10 nucléotides contigus de SEQ ID NO :6 flanquée en 5' de un à plusieurs nucléotides contigus de la séquence SEQ ID NO :7 à partir de son extrémité 3' et/ou flanquée en 3' de un à plusieurs nucléotides contigus de la séquence SEQ ID NO :8 à partir de son extrémité 5'.

Encore préférentiellement, l'aptamère selon l'invention comprend SEQ ID NO :6 flanquée en 3' de un à plusieurs nucléotides contigus de la séquence SEQ ID NO :7 à partir de son extrémité 5' et/ou flanquée en 5' de un à plusieurs nucléotides contigus de la séquence SEQ ID NO :8 à partir de son extrémité 3'.

On entend par « un ou plusieurs » tous les nucléotides, de préférence 3 à 10, de manière particulièrement préférée 5 nucléotides.

On peut citer par exemple les séquences :

- SEQ ID NO:10 : *TCGAA*CGCAATGGGCGCAGATTGGAAGGCCTAGCA*TTGAG* comprenant SEQ ID NO :6 flanquée en 5' des 5 nucléotides contigus de l'extrémité 3' de SEQ ID NO :7 et flanquée en 3' de 5 nucléotides contigus de l'extrémité 5' de SEQ ID NO :8,
- SEQ ID NO:11 : *GTCGAA*CGCAATGGGCGCAGATTGGAAGGCCTAGCA comprenant SEQ ID NO :6 flanquée en 5' des 6 nucléotides contigus de l'extrémité 3' de SEQ ID NO :7.

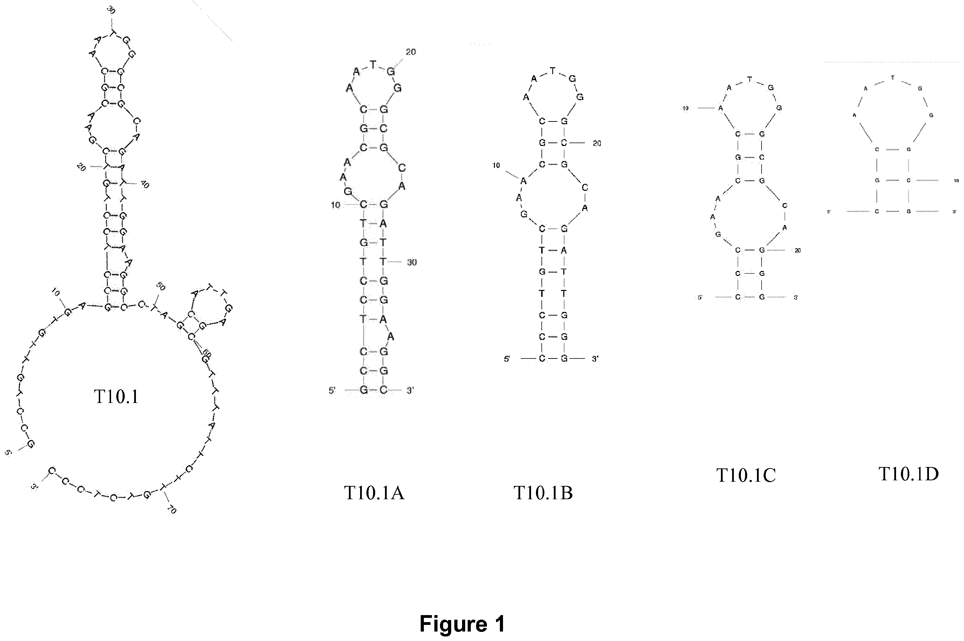

L'aptamère selon l'invention peut également comprendre une séquence dérivée de T10.1C (SEQ ID NO :4) de même structure secondaire tige-boucle-tige-boucle apicale (voir Figure 1), chaque tige étant constituée d'appariements G-C, C-G, A-T ou T-A, chaque boucle comprenant 2 à 10 nucléotides.

Cette séquence peut également se traduire de la façon suivante : $X_1$---$X_n X_{(n+1)}$---$X_m X_{(m+1)}$---$X_p X_{(p+1)}$---$X_r X_{(r+1)}$---$X_o X_{(o+1)}$---$X_s X_{(s+1)}$---$X_t$ dans laquelle chaque X est un nucléotide

$$1 \leq n \leq 5$$

$$n+1 \leq m \leq n+5, \text{ de préférence } n+1 \leq m \leq n+3$$

$$m+1 \leq p \leq m+5$$

$$p+1 \leq r \leq p+10, \text{ de préférence } p+2 \leq r \leq p+8, \text{ de manière particulièrement préférée } r=p+5$$

$$r+1 \leq o \leq r+5$$

$$o+1 \leq s \leq o+5$$

$$s+1 \leq t \leq s+5$$

$$t-s=n$$

o-r=p-m

n, m, p, r, o, s, t sont des entiers et signifient la position du nucléotide,

les nucléotides $X_1$ à $X_n$ et $X_{(s+1)}$ à $X_t$ sont A, T, G ou C, de préférence G ou C, à condition qu'ils s'apparient donc forment une tige,

les nucléotides $X_{(m+1)}$ à $X_p$ et $X_{(r+1)}$ à $X_o$ sont A, T, G ou C de préférence G ou C, à condition qu'ils s'apparient donc forment une tige,

les nucléotides $X_{(n+1)}$ à $X_m$ et $X_{(o+1)}$ à $X_s$ sont A, T, G ou C et ne s'apparient pas donc forment une boucle,

les nucléotides $X_{(p+1)}$ à $X_r$ sont A, T, G ou C et ne s'apparient pas donc forment une boucle.

On appelle par « tige » une séquence double brin s'appariant parfaitement, par exemple :

G-C          A-T

(suite)

| | |
|---|---|
| C-G | A-T |
| G-C | A-T |
| C-G | A-T |

On appelle par « boucle » un motif au sein d'une séquence double brin sans appariement.

Selon un mode de réalisation particulier de l'invention, l'aptamère selon l'invention comprend une séquence dérivée de T10.1C (SEQ ID NO :4) de même structure secondaire tige-boucle-tige-boucle apicale (voir Figure 1), chaque tige étant constituée de 3 appariements G-C, C-G, A-T ou T-A, chaque boucle comprenant 5 nucléotides.

Cette séquence SEQ ID NO :12 peut également se traduire de la façon suivante :

$X_1$---$X_n X_{(n+1)}$---$X_m X_{(m+1)}$---$X_p X_{(p+1)}$---$X_r X_{(r+1)}$---$X_o X_{(o+1)}$---$X_s X_{(s+1)}$---$X_t$ dans laquelle chaque X est un nucléotide

$$n=3$$

$$m=n+3$$

$$p=m+3$$

$$r=p+5$$

$$o=r+3$$

$$s=o+2$$

$$t=s+3$$

n, m, p, r, o, s, t sont des entiers et signifient la position du nucléotide,

les nucléotides $X_1$ à $X_n$ et $X_{(s+1)}$ à $X_t$ sont A, T, G ou C, de préférence G ou C, à condition qu'ils s'apparient donc forment une tige,

les nucléotides $X_{(m+1)}$ à $X_p$ et $X_{(r+1)}$ à $X_o$ sont A, T, G ou C de préférence G ou C, à condition qu'ils s'apparient donc forment une tige,

les nucléotides $X_{(n+1)}$ à $X_m$ et $X_{(o+1)}$ à $X_s$ sont A, T, G ou C et ne s'apparient pas donc forment une boucle,

les nucléotides $X_{(p+1)}$ à $X_r$ sont A, T, G ou C et ne s'apparient pas donc forment une boucle.

On appelle par « tige » une séquence double brin s'appariant parfaitement, par exemple :

| | |
|---|---|
| G-C | A-T |
| C-G | A-T |
| G-C | A-T |
| C-G | A-T |

Un autre objet de l'invention concerne l'utilisation d'un aptamère selon l'invention pour inhiber l'activité enzymatique de la tyrosinase.

La tyrosinase transforme la tyrosine en L-DOPA. L'activité enzymatique de la tyrosinase peut être évaluée par mesure de l'apparition du DOPAchrome, produit d'oxydation coloré de la L-DOPA. La cinétique d'apparition de ce produit d'oxydation est suivie par mesure de l'absorbance à 450 nm.

De préférence, l'invention concerne l'utilisation d'un aptamère selon l'invention en tant qu'agent dépigmentant ou éclaircissant de la peau dans une composition cosmétique ou dermatologique.

Un autre objet de l'invention concerne une composition cosmétique ou pharmaceutique comprenant en tant que principe actif au moins un aptamère selon l'invention en une quantité suffisante pour inhiber l'activité enzymatique de la tyrosinase et un ou plusieurs excipients cosmétiquement/pharmaceutiquement acceptables.

De préférence, la composition de l'invention comprend de 0,00001% à 10%, de préférence de 0,00005 à 5 %, de manière encore préférée de 0.001 à 1% d'un ou plusieurs aptamères selon l'invention. D'une manière générale, toute composition de l'invention peut être appliquée sur la peau ou les phanères.

Elle peut se présenter sous toutes les formes galéniques adaptées à une application topique.

La composition de l'invention peut avoir la forme notamment de solutions aqueuses ou huileuses ou de dispersions du type lotion ou sérum, d'émulsions de consistance liquide ou semi-liquide du type lait, obtenues par dispersion d'une phase aqueuse dans une phase siliconée (H/Si), d'une phase grasse dans une phase aqueuse (émulsion huile-dans-eau) ou inversement d'une phase aqueuse dans une phase grasse (émulsion eau-dans-huile), ou de suspensions ou émulsions de consistance molle du type crème ou gel aqueux ou anhydres, ou encore de microcapsules ou microparticules, ou de dispersions vésiculaires de type ionique et/ou non ionique ou de mousses. Ces compositions sont préparées selon les méthodes usuelles. Les quantités des différents constituants des compositions selon l'invention sont celles classiquement utilisées dans les domaines considérés.

Dans le domaine de la cosmétique, ces compositions constituent notamment des crèmes de nettoyage, de protection, de traitement ou de soin pour le visage, pour les mains, pour les pieds, ou pour le corps (par exemple crèmes de jour, crèmes de nuit, crèmes de démaquillage, crèmes de fond de teint, crèmes antisolaires), des fonds de teint fluides, des laits de démaquillage, des laits corporels de protection ou de soin, des laits anti-solaires, des lotions, gels ou mousses pour le soin de la peau, comme des lotions de nettoyage, des lotions anti-solaires, des lotions de bronzage artificiel, des compositions pour le bain, des compositions désodorisantes comprenant un agent bactéricide, des gels ou lotions après-rasage, des crèmes épilatoires.

Les compositions selon l'invention peuvent également consister en des préparations solides pulvérulentes ou non, par exemple sous forme de stick, d'une poudre pressée, des savons ou de pains de nettoyage. Elle peut encore se présenter sous la forme de patchs, de crayons, de pinceaux et d'applicateurs autorisant une application localisée sur les taches du visage ou des mains. Elle peut être utilisée comme produit de soin ou comme produit de maquillage.

Lorsque la composition est une émulsion, la proportion de la phase grasse peut varier d'environ 5 % à 80 % en poids, et de préférence d'environ 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le co-émulsionnant sont présents, dans la composition, en une proportion allant de 0,3 % à 30% en poids, et de préférence de 0,5 % à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

De façon connue, la composition cosmétique ou pharmaceutique de l'invention peut contenir également des adjuvants habituels dans le domaine cosmétique ou pharmaceutique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans les domaines considérés. Ces adjuvants, selon leur nature, peuvent être introduits dans la phase grasse, dans la phase aqueuse, dans les vésicules lipidiques ou dans les nanoparticules. Les quantités de ces différents adjuvants sont celles classiquement utilisées dans le domaine cosmétique ou pharmaceutique, et par exemple varient d'environ 0,01 % à 10 % du poids total de la composition. Ces adjuvants, selon leur nature, peuvent être introduits dans la phase grasse, dans la phase aqueuse et/ou dans les sphérules lipidiques.

Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique). Comme émulsionnants utilisables dans l'invention, on peut citer par exemple le stéarate de glycérol, le polysorbate 60 et le mélange de PEG-6/PEG-32/Glycol Stéarate vendu sous la dénomination de T'efose 63 par la société Gattefosse.

Comme solvants utilisables dans l'invention, on peut citer les alcools inférieurs notamment l'éthanol et l'isopropanol et le propylène glycol.

Comme gélifiants hydrophiles utilisables dans l'invention, on peut citer les polymères carboxyvinyliques (carbomères), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium, la silice hydrophobe, l'éthylcellulose et le polyéthylène.

Une composition de l'invention peut également comprendre un ou plusieurs autres principes actifs, par exemple destinés à inhiber la mélanogenèse.

Lesdits actifs utilisables en association avec les aptamères selon l'invention, utilisés purs ou provenant d'extraits renfermant ces molécules, sont notamment les composés suivants : l'acide ellagique et ses dérivés ; l'hydroquinone ; l'arbutine ; le résorcinol et ses dérivés ; la vitamine C et ses dérivés ; le pantothénate sulfonate et ses dérivés ; l'acide kojique ; des molécules interférant directement ou indirectement avec l'alpha-mélanocyte stimulating hormone (a-MSH) ou son récepteur ou l'hormone adrénocorticotropique (ACTH) ; les polyols tels que la glycérine, le glycol ou le propylène glycol ; les vitamines ; les agents kératolytiques ou desquamants tels que l'acide salicylique et ses dérivés ; les alpha-hydroxyacides tels que l'acide lactique ou l'acide malique, seuls ou greffés les beta-hydroxyacides ; l'acide ascorbique et ses dérivés ; l'acide rétinoïque ; le rétinaldéhyde ; le rétinol et ses dérivés tels que le palmitate, le propionate ou l'acetate, en préparation liposomique ou non ; des agents antiglycations ou antioxydants pris seuls ou en association tels que le tocophérol et ses dérivés, la thiotaurine, l'hypotaurine, l'aminoguanidine, la thiamine pyrophosphate, la pyridoxamine, la lysine, l'histidine, l'arginine, la phénylalanine, la pyridoxine, l'adénosine triphosphate ; les agents anti-inflammatoires tels que le stearyl glycyrrhétinate ; les agents apaisants et leurs mélanges, les filtres solaires chimiques ou physiques tels que le méthoxycinnamate d'octyle, le butylméthoxydibenzoyl-méthane, l'oxyde de titane et l'oxyde de zinc micronisés ; et les acides désoxyribonucléiques ou nucléiques.

La présente invention concerne également l'utilisation d'au moins un aptamère selon l'invention pour la fabrication d'un médicament destiné à être administré de façon simultanée, séparée ou étalée dans le temps en association avec un ou plusieurs autres actifs, par exemple les actifs décrits ci-dessus.

La présente invention a également pour objet l'utilisation d'au moins un aptamère selon l'invention dans une composition cosmétique ou dermatologique pour dépigmenter ou éclaircir la peau, les poils ou les cheveux humains, ou enlever ou atténuer les taches pigmentaires de la peau humaine.

La présente invention a aussi pour objet l'utilisation d'au moins un aptamère selon l'invention dans ou pour la fabrication d'une composition cosmétique ou dermatologique comme inhibiteur de la synthèse des mélanines.

La présente invention concerne aussi l'utilisation d'au moins un aptamère selon l'invention pour moduler l'expression de la tyrosinase.

La présente invention a aussi pour objet l'utilisation d'au moins un aptamère selon l'invention, dans une composition cosmétique dépigmentante ou éclaircissante de la peau humaine.

La présente invention a encore pour objet l'utilisation d'au moins un aptamère selon l'invention pour la fabrication d'une composition dermatologique dépigmentante ou éclaircissante de la peau humaine.

Un autre objet de l'invention concerne l'utilisation cosmétique d'un aptamère selon l'invention, de préférence pour dépigmenter ou éclaircir la peau, les poils et/ou les cheveux.

La présente invention se rapporte également à un procédé de traitement cosmétique ou dermatologique pour contrôler les excès de pigmentation, dépigmenter ou éclaircir la peau humaine consistant à appliquer sur la peau une composition cosmétique ou dermatologique comprenant au moins un aptamère selon l'invention.

La présente invention se rapporte également à l'utilisation d'au moins un aptamère selon l'invention pour la fabrication d'un médicament destiné au traitement par voie topique ou à la prévention des maladies se traduisant par la surexpression de la tyrosinase.

Un autre objet de l'invention concerne un aptamère selon l'invention pour son utilisation dans le traitement ou la prévention des hyperpigmentations comme les hyperpigmentations régionales par hyper-activité mélanocytaire telles que les mélasmas idiopathiques, les hyperpigmentations localisées par hyper-activité et prolifération mélanocytaire bénigne telles que les taches pigmentaires de sénescence (lentigo seniles), les hyperpigmentations accidentelles telles que la photosensibilisation ou l'hyperpigmentation cicatricielle, et pour le traitement de certaines leucodermies telles que le vitiligo.

Un autre objet de l'invention concerne un aptamère selon l'invention en tant que médicament, de préférence destiné à éclaircir ou dépigmenter la peau, les poils et/ou les cheveux.

Un autre objet de l'invention concerne un aptamère selon l'invention, pour son utilisation dans le traitement ou la prévention des hyperpigmentations comme les hyperpigmentations régionales par hyper-activité mélanocytaire telles que les mélasmas idiopathiques, les hyperpigmentations localisées par hyper-activité et prolifération mélanocytaire bénigne telles que les taches pigmentaires de sénescence (lentigo seniles), les hyperpigmentations accidentelles telles que la photosensibilisation ou l'hyperpigmentation cicatricielle, et pour le traitement de certaines leucodermies telles que le vitiligo.

Les aptamères selon l'invention peuvent être utilisés selon l'invention sous forme vectorisée c'est-à-dire liés à un vecteur. Ce type de vecteur est bien connu de l'homme du métier. Il peut s'agir par exemple de résidu cholestérol, de nanoparticules.

Les aptamères selon l'invention peuvent également être utilisés selon l'invention sous forme de dimères de deux aptamères ou d'un conjugué de plusieurs aptamères. La spécification décrit également un procédé de sélection d'un aptamère selon l'invention comprenant les étapes suivantes :

- Sélection dans une banque d'ADN d'aptamères par la méthode SELEX contre la tyrosinase,

- évaluation du potentiel à inhiber l'activité enzymatique de la tyrosinase des aptamères identifiés à l'étape précédente,
- clonage et séquençage de l'aptamère ainsi sélectionné.

**Exemple 1 : Matériel et Méthodes de sélection des aptamères** anti-**tyrosinase**

**Protéines**

La protéine recombinante humaine TYR (tyrosinase) contenant une étiquette GST en N-terminal a été fournie par ABNOVA. L'étiquette polyhistidine-GST a été produite à l'Institut Européen de Chimie et Biologie.

**Oligonucléotides et banque**

Une banque d'ADN et des amorces fournis par Sigma ont été purifiés par HPLC. Les séquences amorces (P3) 5' GGGAGACAAGAATAAACGCTCAA (SEQ ID NO :9) et (P5) 5' GCCTGTTGTGAGCCTCCTGTCGAA (SEQ ID NO :7) ont été utilisées pour l'amplification de la banque contenant une fenêtre de 30 nucléotides au hasard. La séquence 5' ACTGACTGACTGACTGACTA-6C3-GGGAGACAAGAATAAACGCTCAA a été utilisée pour produire du simple brin tel que décrit dans la littérature (Williams KP, Bartel DP. PCR product with strands of unequal length. Nucleic Acids Res. 1995 Oct 25;23(20):4220-1). L'amorce (P3) biotinylée en 5' a été utilisée pour produire des candidats ADN simple brin. Des candidats ADN ont été synthétisés et purifiés par HPLC par Eurogentec (tableau 1). Avant toute expérience, les populations et candidats ADN ont été chauffés à 75°C pendant 5 min, placés sur la glace pendant 5 minutes, et ensuite mis à température ambiante pendant au moins 5 minutes.

**Sélection In vitro**

Avant la sélection, la banque d'ADN (1 nanomole) a été traitée tel que décrit ci-dessus et incubée avec des filtres (HAWP 0,45 $\mu$M, Millipore) deux fois pour le premier et le second tours et une fois pour tous les autres tours en tampon PBS-Mg (154 mM NaCl, 1 mM $KH_2PO_4$, 2,96 mM $Na_2HPO_4.7H_2O$, 1mM $(CH_3COO)2Mg$, pendant 20 min à température ambiante. A chaque tour, une contre-sélection supplémentaire a été réalisée contre l'étiquette polyhistidine-GST. Ensuite, la banque contre-sélectionnée a été mélangée avec la tyrosinase (20 picomoles) pendant 20 minutes et les candidats non liés ont été séparés par la technique de rétention sur filtre. Après la filtration, les candidats liés à la tyrosinase ont été élués par incubation 20 min à 65°C dans 500 $\mu$L de phénol/urée 7M, précipités et amplifiés par PCR pour produire du simple brin utilisé pour le tour suivant de sélection. Réduire la quantité de candidats et de cible pendant la sélection pour atteindre 25 et 1 picomoles au dixième tour, respectivement, a augmenté la rigueur de la sélection. Avant le clonage, les populations de chaque tour de sélection ont été évaluées pour leur capacité à inhiber l'activité tyrosinase.

**Clonage et séquençage**

Les séquences sélectionnées des tours 7 et 10 ont été clonées en utilisant le kit de clonage TOPO TA (Invitrogen) et séquencées en utilisant le kit de séquençage BigDye Terminator v1.1 cycle (Applied Biosystems) selon les instructions du fabricant.

**Caractérisation des Candidats SPR (Surface Plamon Resonance)**

Toutes les expériences ont été réalisées sur un dispositif BIAcore 3000 avec le tampon PBS-Mg à 23°C. Les oligonucléotides biotinylés chauffés à 75°C pendant 5 minutes, placés sur la glace pendant 5 minutes, et ensuite placés à température ambiante pendant au moins 5 minutes, ont été immobilisés sur des puces de détection revêtues de Streptavidine (XanTec bioanalytics). Les oligonucléotides ont été injectés à 100 nM pendant 4 minutes à 5 $\mu$l/min pour atteindre 800 à 1000 RU.

La tyrosinase et la GST-His témoin ont été injectées à 500 nM et 1 $\mu$M à un débit de 20 $\mu$l/min à 23°C. Les mêmes injections ont été réalisées avec du tampon PBS-Mg et le tampon de stockage de la tyrosinase pour soustraire les effets du tampon au sensorgramme spécifique.

Après chaque injection de protéines, la surface fonctionnalisée de la puce a été régénérée avec un mélange de 40% formamide, 3.6 M urée et 30 mM EDTA pour une impulsion de 1 min.

Les données ont été analysées avec le logiciel BIA eval 4.1.

**Exemple 2 : Résultats de la sélection des aptamères anti- tyrosinase**

La procédure de sélection-amplification contre la cible tyrosinase a été répétée 10 fois; les tours SELEX 7 et 10 ont été clonés et séquencés. 41 and 74 candidats ADN des tours 7 et 10, respectivement, ont été analysés. La séquence T10.1 représente 42 % des séquences du tour 10. Les autres séquences montrent une grande diversité. La séquence T10.1 n'était pas présente au tour 7.

La structure secondaire du candidat T10.1 a été prédite en utilisant le logiciel mfold (Zuker, M., 2003. Nucleic Acids Res 31(13), 3406-3415). Une longue tige-boucle imparfaite incluant une partie de l'amorce en 5' P5 (GCCTCCTGTCGAA) et montrant une potentielle boucle interne de 5 nucléotides et une boucle apicale de 5 nucléotides a été prédite (Figure 1).

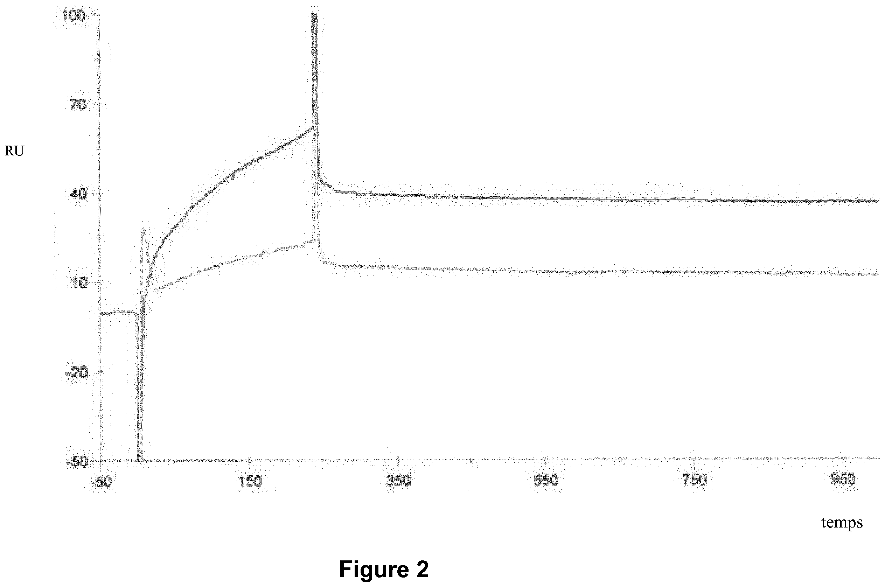

La liaison de ce candidat T10.1 et des versions tronquées dérivées du candidat pleine longueur (tableau 1) a été recherchée par Résonance Plasmonique de Surface (SPR). Environ 1000 RU (unité de résonance) du candidat T10.1 biotinylé ont été immobilisées et la tyrosinase a été injectée à 500 nM ou à 1 μM. Un signal de 16 et 42 RU a été détecté pour 500 nM et 1 μM, respectivement, indiquant une interaction entre le candidat T10.1 et la tyrosinase (Figure 2).

**Exemple 3 : Mesure de l'activité tyrosinase**

**3.1 Principe**

Une enzyme est une protéine capable de catalyser spécifiquement la transformation d'un ou deux substrats.

En prenant un modèle simplifié de réaction enzymatique : $S \xrightarrow{E} P$, la vitesse de réaction s'écrit :

$$v = \frac{d(P)}{dt} = -\frac{d(S)}{dt} .$$

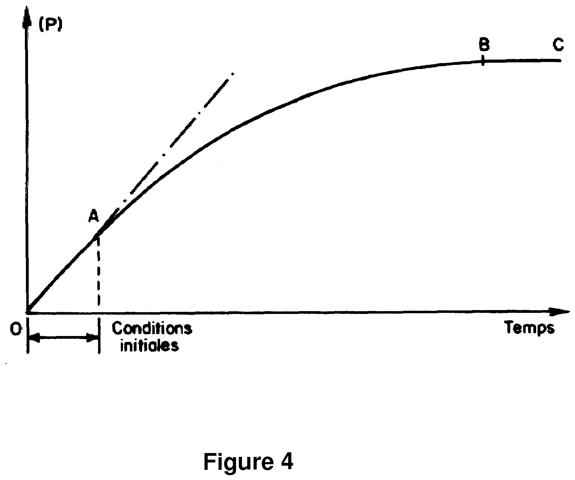

Pour tracer une courbe $(P) = f(t)$, l'enzyme E agit sur le substrat S ; le temps zéro correspond au déclenchement de la réaction. L'apparition du produit P est mesurée en fonction du temps. (voir Figure 4)

$$v = \frac{d(P)}{dt}$$

La vitesse de réaction $v = \frac{d(P)}{dt}$ est constante pendant les conditions initiales (partie de courbe 0A). Pour cette portion de courbe, la tangente à l'origine se confond avec la courbe : la vitesse, pente de la tangente 0A, est appelée vitesse initiale. Puis la vitesse diminue (portion de courbe AB) et s'annule (portion BC). La vitesse s'annule lorsque l'un des substrats est consommé ou lorsqu'il s'établit un équilibre.

Lorsque la vitesse d'une réaction enzymatique est déterminée, c'est toujours la vitesse initiale qui est calculée. Les mesures de vitesse sont donc faites dans les conditions initiales où moins de 10 % de la quantité de substrat est hydrolysé. Tant que $[S]>>[E]$, la vitesse initiale est proportionnelle à la concentration de l'enzyme : elle traduit donc l'activité d'une préparation de l'enzyme exprimée en unités enzymatiques.

L'activité enzymatique de la tyrosinase est mesurée par détection du produit coloré d'oxydation de son produit. Le produit de la tyrosinase, la L-DOPA, est oxydé en un produit coloré, le DOPAChrome. La cinétique d'apparition de ce produit d'oxydation successif de la L-DOPA puis de la L-DOPAQuinone est suivie par mesure de l'absorbance à 450nm.

**3.2 Mesure de l'activité enzymatique de la tyrosinase dans une culture de mélanocytes**

Des mélanocytes humains normaux sont ensemencés en microplaques (96 puits) à raison de $1.10^4$ cellules par puits. Après 72h de traitement par les différents candidats du Tour 10 et issus du Tour 10 (T10.1, T.10.1A, T10.1B, T10.1C, T10.1D), l'activité enzymatique de la tyrosinase est déterminée. Chaque aptamère est testé à 4 doses croissantes : 4, 20, 100 et 200 nM.

Les cellules sont rincées au PBS puis 50 μL de Triton X100 (0,5%) sont ajoutés dans chaque puits et la plaque est mise sous agitation pendant une heure à 4°C. La réaction est initiée en ajoutant 50 μL de substrat (L-DOPA 10 mM, Sigma) à chaque puits. L'apparition du DOPAchrome est mesurée toutes les 2 minutes à 450 nm pendant une heure et à 37°C, sous agitation régulière. Les vitesses de réaction ainsi obtenues sont exprimées en Unité DO/min.

Les variations d'absorbance (450 nm) au cours du temps sont suivies à l'aide d'un spectrophotomètre lecteur de plaque BMG Polarstar à +20°C. La courbe représentant l'absorbance (en unités d'absorbance) en fonction du temps (en minutes) est tracée.

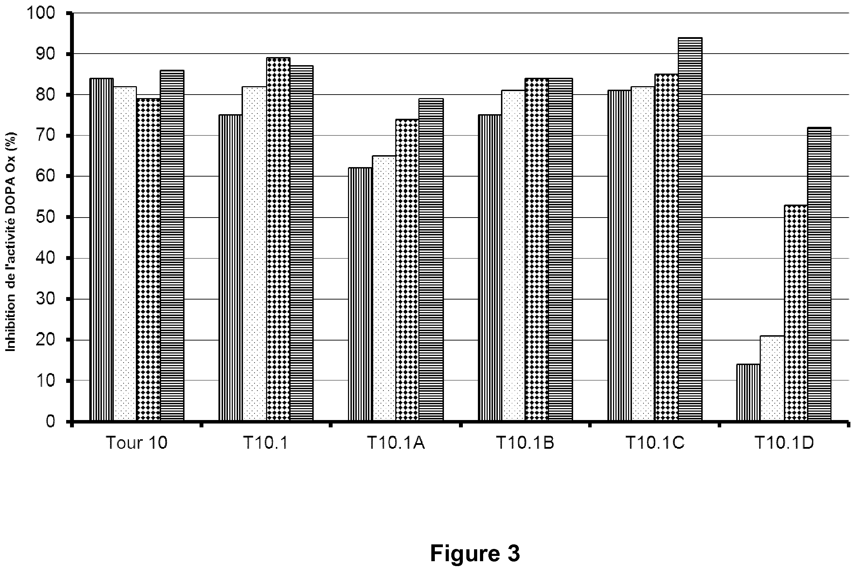

La vitesse initiale de la réaction est déterminée par le calcul de la pente de la tangente à l'origine. $V_0$ représente la vitesse initiale en absence d'effecteur et $V_i$ cette vitesse en présence d'un effecteur. Le rapport $(V_i/V_0) \times 100$ est calculé : si ce rapport est égal à 100, l'effecteur n'a pas d'effet sur l'activité de l'enzyme ; s'il est inférieur à 100, l'effecteur est un inhibiteur de l'activité de l'enzyme ; s'il est supérieur à 100, il s'agit d'un activateur. Les résultats de cette mesure sont présentés figure 3.

Les résultats présentés en figure 3 mettent en évidence un effet inhibiteur de chacun des candidats testés sur l'activité de la tyrosinase. Cet effet est significatif pour chacune des conditions testées, à l'exception des concentrations les plus faibles du candidat le plus raccourci, T10.1D (4 et 20nM).

L'ensemble des candidats constituant le Tour 10 présente un effet significatif supérieur à 75%, sur l'activité de la tyrosinase, quelle que soit la concentration testée. Aucun effet dose n'est observé. Des résultats similaires sont obtenus pour les candidats T10.1.

Les candidats raccourcis T10.1A et T10.1B sont tous également inhibiteurs de l'activité tyrosinase. Cependant cette inhibition est inférieure à celle du candidat pleine taille du Tour 10 et T10.1, en particulier pour les concentrations les plus faibles (4 et 20nM).

Le candidat T10.1C présente un très bon pouvoir inhibiteur de cet enzyme et ceux, quelque soit la dose testée. On note en particulier, une inhibition de près de 95% de l'activité de la tyrosinase, pour ce candidat lorsqu'il est utilisé à la concentration de 200nM. Aucun autre candidat testé ne présente une activité aussi importante à cette concentration.

Le candidat le plus raccourci, T10.1D, présente la plus faible activité inhibitrice, quelle que soit la dose testée.

Ces résultats suggèrent l'absence dans la structure de l'aptamère T10.1D, d'une région importante à l'activité inhibitrice vis-à-vis de la tyrosinase.

## EXEMPLES DE FORMULATIONS

### Exemple A : Poudre cosmétique pour l'éclaircissement du teint du visage

| Microcellulose | 20,00% |
|---|---|
| Sodium lauryl sulfoacetate | 15,00% |
| Aptamère selon l'invention | 1% |
| Parfum, colorants, conservateurs | qs. |
| Talc | Qsp. 100% |

Cette poudre présente une double action. Elle permet un nettoyage de la peau, et de plus elle permet, par un usage régulier durant quelques jours, d'éclaircir le teint. On peut l'appliquer sur la peau du visage une à deux fois par jour.

### Exemple B : Crème cosmétique de jour dépigmentante sous forme d'émulsion-gel

| Glycérine | 5,00% |
|---|---|
| triglycérides caprylique/caprique/succinique | 5,00% |
| Méthoxycinnamate d'octyle | 1,00% |
| Diméthicone copolyol | 0,50% |
| Acrylates / C10-30 alkyl acrylate crosspolymer | 0,50% |
| Aptamère selon l'invention | 1% |
| Neutralisant | qs. |
| Conservateurs, parfum, colorants | qs. |
| Eau | qsp. 100% |

Certaines personnes soumises au rayonnement plus ou moins intense de la lumière du jour, voire du soleil directement, souhaitent conserver un teint clair et éviter l'apparition de taches pigmentées. L'utilisation de l'émulsion-gel ci-dessus permettra d'atteindre ce but. Cette composition s'applique sur le visage généralement le matin. Elle agit aussi bien de manière préventive que curative sur la pigmentation, régulière ou non, du visage.

**Exemple C : Composition cosmétique fluide protectrice du rayonnement solaire (SPF 30)**

| | |
|---|---|
| Pentacyclométhicone volatile | 49,00% |
| Dioxyde de titane | 15,00% |
| Méthoxycinnamate d'octyle | 7,50% |
| Glycérine | 5,00% |
| Phényltriméthicone | 5,00% |
| Diméthicone copolyol | 3,00% |
| Polyméthylméthacrylate | 2,50% |
| Butyl méthoxydibenzoyle méthane | 1,00% |
| Aptamère selon l'invention | 1% |
| Neutralisant, Parfum, Conservateurs, antioxydants | qs. |
| Eau | qsp. 100% |

Cette composition est à utiliser avant l'exposition à un rayonnement solaire intense. Elle prévient l'apparition de taches pigmentaires, chez les personnes prédisposées à ce phénomène,. Il est à noter que la présence d'une concentration élevée en filtre solaire permet de compenser la diminution de la protection naturelle, conséquence de la baisse du taux de mélanine.

**Exemple D : Crème dermatologique pour le traitement d'hyperpigmentations cutanées d'origine pathologique ou traumatique**

| | |
|---|---|
| Glycéryl stéarate + Peg-100 stéarate | 5,00% |
| Polyisobutène hydrogéné | 4,00% |
| Magnésium ascorbyl phosphate | 3,00% |
| Tricaprylate /caprate de glycérol | 3,00% |
| Squalane | 3,00% |
| Glycérine | 2,00% |
| Cire d'abeille | 1,50% |
| Cétéaryl octanoate | 1,50% |
| Alcool cétylique | 1,00% |
| Alcool stéarylique | 1,00% |
| Diméthicone | 1,00% |
| Gomme xanthane | 0,30% |
| Acide éthylène diamine tétracétique | 0,20% |
| Acide citrique | 0,10% |
| Citrate de sodium | 0,10% |
| Aptamère selon l'invention | 1% |
| Neutralisant, Parfum, Conservateurs | qs. |
| Eau | qsp. 100% |

L'utilisation de cette crème permet d'atténuer les hyperpigmentations cutanées d'origine pathologique ou traumatique. Cette crème permet également d'atténuer les contrastes de couleur à la périphérie des zones dépigmentées en cas de vitiligo.

**Exemple E : Lotion cosmétique visage pour éclaircir le teint**

| | |
|---|---|
| Alcool éthylique | 30,00% |
| PPG-3 Myristyl éther | 5,00% |
| Glycérine | 2,00% |
| Carbomer | 0,20% |
| Polysorbate 20 | 0,20% |
| Aptamère selon l'invention | 1% |
| Neutralisant, Parfum, Conservateurs | qs. |
| Eau | qsp. 100% |

Cette lotion pour éclaircir le teint s'utilise après le démaquillage et le nettoyage de la peau.

**Exemple F : Sérum cosmétique éclaircissant pour le visage**

| | |
|---|---|
| Eau | qsp 100% |
| Glycerine | 2% |
| EDTA tetrasodique<br>Acide citrique<br>Citrate trisodique | qsp pH souhaité |
| Gomme xanthane | 0.25% |
| Polyacrylamide, C13.14 isoparaffin, laureth-7 | 0.5% |
| Dimethicone copolyol | 0.25% |
| Aptamère selon l'invention | 1% |
| Parfum, colorant, conservateur | qs |

Une goutte de cette composition très concentrée de sérum, s'applique sur le visage généralement avant l'application d'une crème pour le visage. Ce sérum s'utilise habituellement par cures d'une à deux semaines pour obtenir ou entretenir un éclaircissement du teint.

**Exemple G : Lotion cosmétique pour éclaircir la pilosité corporelle**

| | |
|---|---|
| Eau | qsp 100% |
| Alcool | 50% |
| Panthenylethyl ether | 0.5% |
| Acétate DL-$\alpha$-tocopherol | 0.2% |
| Polysorbate 60 | 1% |

(suite)

| Aptamère selon l'invention | 1% |
|---|---|
| Parfum | 0.2% |
| Glycerine | 0.5% |
| Colorant | qs |

Cette lotion s'applique sur les zones pileuses à éclaircir, notamment les bras, pendant la durée suffisante pour obtenir un éclaircissement progressif des poils.

**Exemple H : Gel crème cosmétique anti-taches pour les mains**

| Caprilic/capric diglyceryl succinate | 6% |
|---|---|
| Octyl octanoate | 2.5% |
| Methoxycinnamate d'octyle | 6% |
| Aptamère selon l'invention | 1% |
| Phenyltriméticone | 2.5% |
| Benzophenon-3 | 0.5% |
| Hyaluronate de sodium | 0.05% |
| Gomme xanthane | 0.2% |
| Acrylates/C10.30 alkyl acrylate copolymer | 0.5% |
| Glycerine | 2% |
| PEG 150 | 3% |
| Neutralisants, Colorants, parfum, conservateurs | qs |
| Eau purifiée | qsp 100% |

Cette crème doit être appliquée directement sur les taches (lentigos solaires et/ou séniles) des mains, pour en atténuer la coloration.

## Revendications

1. Aptamère d'ADN comprenant la séquence SEQ ID NO : 5 capable d'inhiber l'activité enzymatique de la tyrosinase de conversion de la tyrosine en L-DOPA et dopaquinone.

2. Aptamère selon la revendication 1, capable de se lier avec une forte affinité à la tyrosinase.

3. Aptamère selon la revendication 1 ou 2, résistant aux nucléases.

4. Aptamère selon l'une quelconque des revendications 1 à 3, comprenant une séquence choisie dans le groupe consistant en SEQ ID NO :1, SEQ ID NO :2, SEQ ID NO :3, SEQ ID NO :4 et SEQ ID NO :6.

5. Aptamère selon la revendication 4, comprenant la séquence SEQ ID NO :4.

6. Aptamère selon la revendication 4, comprenant au moins 10 nucléotides contigus de SEQ ID NO :6 flanquée en 5' de un à plusieurs nucléotides contigus de la séquence SEQ ID NO :7 à partir de son extrémité 3' et/ou flanquée en 3' de un à plusieurs nucléotides contigus de la séquence SEQ ID NO :8 à partir de son extrémité 5'.

7. Aptamère selon la revendication 6, comprenant SEQ ID NO :6 flanquée en 5' de un à plusieurs nucléotides contigus de la séquence SEQ ID NO :7 à partir de son extrémité 3' et/ou flanquée en 3' de un à plusieurs nucléotides contigus de la séquence SEQ ID NO :8 à partir de son extrémité 5'.

8. Aptamère selon l'une quelconque des revendications 1 à 3, comprenant la séquence suivante :

$$X_1 \text{---} X_n X_{(n+1)} \text{---} X_m X_{(m+1)} \text{---} X_p X_{(p+1)} \text{---} X_r X_{(r+1)} \text{---} X_o X_{(o+1)} \text{---} X_s X_{(s+1)} \text{---} X_t$$

dans laquelle chaque X est un nucléotide

$$1 \leq n \leq 5$$

$$n+1 \leq m \leq n+5$$

$$m+1 \leq p \leq m+5$$

$$p+1 \leq r \leq p+10, \text{ de préférence } p+2 \leq r \leq p+8, \text{ de manière particulièrement préférée } r=p+5$$

$$r+1 \leq o \leq r+5$$

$$o+1 \leq s \leq o+5$$

$$s+1 \leq t \leq s+5$$

$$t-s=n$$

$$o-r=p-m$$

$n, m, p, r, o, s, t$ sont des entiers et signifient la position du nucléotide,
les nucléotides $X_1$ à $X_n$ et $X_{(s+1)}$ à $X_t$ sont A, T, G ou C, de préférence G ou C, à condition qu'ils s'apparient donc forment une tige,
les nucléotides $X_{(m+1)}$ à $X_p$ et $X_{(r+1)}$ à $X_o$ sont A, T, G ou C de préférence G ou C, à condition qu'ils s'apparient donc forment une tige,
les nucléotides $X_{(n+1)}$ à $X_m$ et $X_{(o+1)}$ à $X_s$ sont A, T, G ou C et ne s'apparient pas donc forment une boucle,
les nucléotides $X_{(p+1)}$ à $X_r$ sont A, T, G ou C et ne s'apparient pas donc forment une boucle.

9. Aptamère selon l'une quelconque des revendications précédentes, pour son utilisation comme inhibiteur de l'activité enzymatique de la tyrosinase.

10. Composition cosmétique ou pharmaceutique comprenant en tant que principe actif un aptamère selon l'une quelconque des revendications 1 à 8 en une quantité suffisante pour inhiber l'activité enzymatique de la tyrosinase et un ou plusieurs excipients cosmétiquement/pharmaceutiquement acceptables.

11. Composition selon la revendication 10, **caractérisée en ce qu'**elle comprend de 0,00001 % à 10%, de préférence de 0,00005 à 5 %, de manière encore préférée de 0,001 à 1% en poids de la composition d'un ou plusieurs aptamères

12. Utilisation cosmétique d'un aptamère selon l'une quelconque des revendications 1 à 8, de préférence pour dépigmenter ou éclaircir la peau, les poils et/ou les cheveux.

13. Aptamère selon l'une quelconque des revendications 1 à 8 en tant que médicament.

14. Aptamère selon l'une quelconque des revendications 1 à 8, pour son utilisation dans le traitement ou la prévention des hyperpigmentations régionales par hyper-activité mélanocytaire telles que les mélasmas idiopathiques, des hyperpigmentations localisées par hyper-activité et prolifération mélanocytaire bénigne telles que les taches pigmentaires de sénescence (lentigo seniles), des hyperpigmentations accidentelles telles que la photosensibilisation ou l'hyperpigmentation cicatricielle, et pour le traitement de certaines leucodermies telles que le vitiligo.

**Patentansprüche**

1. DNA-Aptamer, das die Sequenz SEQ ID Nr.: 5 umfasst, das in der Lage ist, die enzymatische Aktivität von Tyrosinase zur Umwandlung von Tyrosin in L-DOPA und Dopaquinon zu hemmen.

**2.** Aptamer nach Anspruch 1, das in der Lage ist, sich mit starker Affinität an die Tyrosinase zu binden.

**3.** Aptamer nach Anspruch 1 oder 2, das gegen Nukleasen resistent ist.

**4.** Aptamer nach einem der Ansprüche 1 bis 3, das eine Sequenz umfasst, die in der Gruppe ausgewählt ist, die aus der SEQ ID Nr.:1, SEQ ID Nr.:2, SEQ ID Nr.:3, SEQ ID Nr.:4 und SEQ ID Nr:6 besteht.

**5.** Aptamer nach Anspruch 4, das die SEQ ID Nr.:4 umfasst.

**6.** Aptamer nach Anspruch 4, das mindestens 10 benachbarte Nukleotide der SEQ ID Nr.:6 umfasst, die ausgehend von ihrem 3'-Ende von einem bis mehreren benachbarten Nukleotiden der Sequenz SEQ ID Nr.:7 5'-flankiert ist und/oder ausgehend von ihrem 5'-Ende von einem bis mehreren angrenzenden Nukleotiden der Sequenz SEQ ID Nr.:8 3'-flankiert ist.

**7.** Aptamer nach Anspruch 6, das die SEQ ID Nr.:6 umfasst, die ausgehend von ihrem 3'-Ende von einem bis mehreren angrenzenden Nukleotiden der Sequenz SEQ ID Nr.:7 5'-flankiert ist und/oder ausgehend von ihrem 5'-Ende von einem bis mehreren angrenzenden Nukleotiden der Sequenz SEQ ID Nr.:8 3'-flankiert ist.

**8.** Aptamer nach einem der Ansprüche 1 bis 3, das die folgende Sequenz umfasst:

$$X_1\text{---}X_nX_{(n+1)}\text{---}X_mX_{(m+1)}\text{---}X_pX_{(p+1)}\text{---}X_rX_{(r+1)}\text{----}X_oX_{(o+1)}\text{---}X_sX_{(s+1)}\text{---}X_t$$

wobei jedes X ein Nukleotid ist

$$1 \leq n \leq 5$$

$$n+1 \leq m \leq n+5$$

$$m+1 \leq p \leq m+5$$

$$p+1 \leq r \leq p+10, \text{ vorzugsweise } p+2 \leq r \leq p+8, \text{ vorzugsweise } r = p+5$$

$$r+1 \leq 0 \leq r+5$$

$$o+1 \leq s \leq o+5$$

$$s+1 \leq t \leq s+5$$

$$t-s = n$$

$$o-r = p-m$$

n, m, p, r, o, s, t ganze Zahlen sind und die Position des Nukleotids ausdrücken,
die Nukleotide $X_1$ bis $X_n$ und $X_{(s+1)}$ bis $X_t$ A, T, G oder C, vorzugsweise G oder C sind, unter der Bedingung, dass sie sich paaren und somit einen Stamm bilden,
die Nukleotide $X_{(m+1)}$ bis $X_p$ und $X_{(r+1)}$ bis $X_o$ A, T, G oder C, vorzugsweise G oder C sind, unter der Bedingung, dass sie sich paaren und somit einen Stamm bilden,
die Nukleotide $X_{(n+1)}$ bis $X_m$ und $X_{(o+1)}$ bis $X_s$ A, T, G oder C sind und sich nicht paaren und somit eine Schleife bilden,
die Nukleotide $X_{(p+1)}$ bis $X_r$ A, T, G oder C sind und sich nicht paaren und somit eine Schleife bilden.

9. Aptamer nach einem der vorhergehenden Ansprüche für seine Verwendung als Hemmstoff für die enzymatische Aktivität von Tyrosinase.

10. Kosmetische oder pharmazeutische Zusammensetzung, die als Wirkstoff ein Aptamer nach einem der Ansprüche 1 bis 8 in einer Menge, die ausreicht, um die enzymatische Aktivität von Tyrosinase zu hemmen, und einen oder mehrere kosmetisch/pharmazeutisch annehmbare Träger umfasst.

11. Zusammensetzung nach Anspruch 10, **dadurch gekennzeichnet, dass** sie von 0,00001 bis 10, vorzugsweise von 0,00005 bis 5, noch mehr zu bevorzugen von 0,001 bis 1 Gew.-% der Zusammensetzung ein oder mehrere Aptamere enthält.

12. Kosmetische Verwendung eines Aptamers nach einem der Ansprüche 1 bis 8, vorzugsweise zum Depigmentieren oder Aufhellen der Haut, der Körperhaare und/oder der Haare.

13. Aptamer nach einem der Ansprüche 1 bis 8 als Medikament.

14. Aptamer nach einem der Ansprüche 1 bis 8 für seine Verwendung bei der Behandlung oder Prävention von regionalen Hyperpigmentierungen durch melanozytäre Hyperaktivität, wie idiopathischen Melasmen, lokalisierten Hyperpigmentierungen durch Hyperaktivität und gutartige melanozytäre Proliferation, wie Alterspigmentflecken (lentigo seniles), akzidentellen Hyperpigmentierungen, wie Photosensibilisierung oder Narbenhyperpigmentierung, und für die Behandlung von bestimmten Leukodermien, wie Vitiligo.

**Claims**

1. DNA aptamer comprising the sequence SEQ ID NO:5 capable of inhibiting the enzymatic activity of tyrosinase for the conversion of tyrosine into L-DOPA and dopaquinone.

2. Aptamer according to claim 1, capable of binding with high affinity to tyrosinase.

3. Aptamer according to claim 1 or 2, that is resistant to nucleases.

4. Aptamer according to any one of claims 1 to 3, comprising a sequence selected from the group consisting of SEQ ID NO:1, SEQ ID NO:2, SEQ ID NO:3, SEQ ID NO:4 and SEQ ID NO:6.

5. Aptamer according to claim 4, comprising the sequence SEQ ID NO:4.

6. Aptamer according to claim 4, comprising at least 10 contiguous nucleotides of SEQ ID NO:6 flanked in 5' by one or more contiguous nucleotides of the sequence SEQ ID NO:7 starting from its 3' end and/or flanked in 3' by one or more contiguous nucleotides of the sequence SEQ ID NO:8 starting from its 5' end.

7. Aptamer according to claim 6, comprising SEQ ID NO:6 flanked in 5' by one or more contiguous nucleotides of the sequence SEQ ID NO:7 starting from its 3' end and/or flanked in 3' by one or more contiguous nucleotides of the sequence SEQ ID NO:8 starting from its 5' end.

8. Aptamer according to any one of claims 1 to 3, comprising the following sequence:

$$X_1 \text{---} X_n X_{(n+1)} \text{---} X_m X_{(m+1)} \text{---} X_p X_{(p+1)} \text{---} X_r X_{(r+1)} \text{---} X_o X_{(o+1)} \text{---} X_s X_{(s+1)} \text{---} X_t$$

wherein each X is a nucleotide

$$1 \leq n \leq 5$$

$$n+1 \leq m \leq n+5$$

$$m+1 \leq p \leq m+5$$

$$p+1 \leq r \leq p+10, \quad \text{preferably} \quad p+2 \leq r \leq p+8, \quad \text{in} \quad \text{a}$$
particularly preferred manner $r=p+5$

$$r+1 \leq o \leq r+5$$

$$o+1 \leq s \leq o+5$$

$$s+1 \leq t \leq s+5$$

$$t-s=n$$

$$o-r=p-m$$

n, m, p, r, o, s, t are integers and signify the position of the nucleotide,
nucleotides $X_1$ to $X_n$ and $X_{(s+1)}$ to $X_t$ are A, T, G or C, preferably G or C, provided that they are paired and thus form a stem,
nucleotides $X_{(m+1)}$ to $X_p$ and $X_{(r+1)}$ to $X_o$ are A, T, G or C, preferably G or C, provided that they are paired and thus form a stem,
nucleotides $X_{(n+1)}$ to $X_m$ and $X_{(o+1)}$ to $X_s$ are A, T, G or C and are unpaired and thus form a loop,
nucleotides $X_{(p+1)}$ to $X_r$ are A, T, G or C and are unpaired and thus form a loop.

9. Aptamer according to any one of the preceding claims, for use as inhibitor of the enzymatic activity of tyrosinase.

10. Cosmetic or pharmaceutical composition comprising as active agent an aptamer according to any one of claims 1 to 8 in an amount sufficient to inhibit the enzyme activity of tyrosinase and one or more cosmetically/pharmaceutically acceptable excipients.

11. Composition according to claim 10, **characterized in that** it comprises from 0.00001% to 10%, preferably from 0.00005% to 5%, more preferably from 0.001% to 1% by weight of the composition, of one or more aptamers.

12. Cosmetic use of an aptamer according to any one of claims 1 to 8, preferably for depigmenting or lightening the skin, body hair and/or head hair.

13. Aptamer according to any one of claims 1 to 8 as medication.

14. Aptamer according to any one of claims 1 to 8, for use in the treatment or prevention of regional hyperpigmentations due to melanocyte hyperactivity such as idiopathic melasmas, localized hyperpigmentations due to benign melanocyte hyperactivity and proliferation such as age or liver spots (senile lentigines), accidental hyperpigmentations such as photosensitization or hyperpigmentation related to scarring, and in the treatment of certain leukodermas such as vitiligo.

Figure 1

**Figure 2**

EP 2 935 587 B1

**Figure 3**

Inhibition de l'activité DOPA Ox (%)

Tour 10 · T10.1 · T10.1A · T10.1B · T10.1C · T10.1D

**Figure 4**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

*   WO 9119813 A

**Littérature non-brevet citée dans la description**

*   **M TAREQ HASSAN KHAN et al.** *Minerva Biotecnologica,* Décembre 2006, vol. 18, 171-179
*   **WILLIAMS KP ; BARTEL DP.** PCR product with strands of unequal length. *Nucleic Acids Res.,* 25 Octobre 1995, vol. 23 (20), 4220-1
*   **ZUKER, M.** *Nucleic Acids Res,* 2003, vol. 31 (13), 3406-3415